# EUROPEAN PATENT SPECIFICATION

(11) **EP 1 343 488 B1**
(45) Date of publication and mention of the grant of the patent: **15.08.2007**
(21) Application number: 01998341.0
(22) Date of filing: 29.11.2001
(51) Int. Cl.: A61K 31/122, A61K 31/7072, A61P 29/00, A61P 9/10, A61P 43/00

(54) **TREATMENT OF STATIN SIDE EFFECTS**
BEHANDLUNG DER NEBENWIRKUNGEN VON STATINEN
TRAITEMENT DES EFFETS SECONDAIRES DE LA STATINE

(30) Priority: 29.11.2000 AU PR177300
(43) Date of publication of application: 17.09.2003
(73) Proprietor: Magral Limited, Melbourne, Victoria 3004 (AU)
(72) Inventor: LINNANE, Anthony, William, Canterbury, Victoria 3126 (AU)
(74) Representative: Jones, Helen M.M.
(86) International application number: PCT/AU2001/001545
(87) International publication number: WO 2002/043721

(56) References cited:
- EP-A- 0 383 432
- WO-A-01/74361
- WO-A-01/85178
- WO-A-93/16704
- BE-A- 1 005 939
- DE-A- 2 462 312
- US-A- 4 465 669
- US-A- 4 929 437
- US-A- 5 316 765
- US-A- 5 973 224
- DATABASE WPI Section Ch, Week 198345 Derwent Publications Ltd., London, GB; Class B03, AN 1983-813471 XP002311568 & SU 988 814 A (ORDZHONIKIDZE CHEM-PHARM) 15 January 1983 (1983-01-15)

## Description

### FIELD OF THE INVENTION

The present invention relates generally to treatment of muscle pain and/or fatigue and to methods for treatment of side effects of statin therapy. In particular, the invention relates to the use of certain substituted benzoquinones, eg Coenzymes Q, particularly Coenzyme Q10 (Q10) in combinative therapy with other agents such as uridine, its salts, esters, tautomers or certain biological precursors thereof ("uridine related compounds"). The invention is also directed to compositions, uses and combination packs or kits related to the treatment methods.

### BACKGROUND TO THE INVENTION

There are numerous drug therapies such as AZT, corticosteroids, cancer chemotherapeutic agents and hypercholesterolemic drugs, which are known to give rise to potentially serious side effects. These effects can be disabling and may last for the duration of the causative drug treatment or even after the drug treatment is complete, affecting not only an individual's capacity to work but also perform the simple tasks involved in day to day life. One particular group of drugs for which side effects are well recognised is the statins which are commonly used to treat hypercholesterolemia, a major cause of cardiovascular disease.

Cardiovascular disease is a term that encompasses a broad range of diseases and syndromes relating to the impairment of function of the heart and its associated network of blood vessels within the body. In spite of decades of declining death rate in the developed world, cardiovascular disease is still the single most common cause of death accounting for about one third of all deaths in the United States in 1997. Cardiovascular disease has many causes and is characterised by complex interactions between the heart, blood vessels, peripheral organs and the tissues. Some types of cardiovascular disease such as coronary heart disease or stroke can occur acutely and without warning, often with severe consequences, including death. Medically these are managed with aggressive treatment (drugs and surgery) followed by chronic treatment to prevent recurrence. Other types of cardiovascular disease such as hypertension (high blood pressure) and hyperlipidemia (high cholesterol) progress slowly, often without overt symptoms, and must be managed by diet and long-term chronic drug therapy.

Although cholesterol is an essential component of a healthy functioning body, being required for the formation of functional membranes, steroid hormones and bile acids, excessive levels, particularly when associated with low density lipoproteins (LDLs), constitute a health risk. It is well established that there is a cause and effect relationship between hypercholesterolemia (excessive blood cholesterol levels) and disease and mortality from coronary artery (heart) disease. Of the deaths resulting from cardiovascular disease, more than three quarters can be attributed to atherosclerosis and its complications.

Atherosclerosis is a generalized disease of the arteries that develops in a symptom free manner over many years. The most common outcome of atherosclerosis is coronary heart disease, followed by stroke and peripheral vascular disease. Elevated blood cholesterol concentration is a major contributing factor in the development of atherosclerosis. In situations of excessive blood cholesterol levels, cholesterol is gradually deposited on the artery walls together with other fats, resulting in build up which disrupts the free flow of blood, with potentially severe results. To lower high cholesterol levels, patients are treated with a range of drugs, commonly known as the statins, which include atorvastatin, simvastatin, pravastatin, lovastatin, cerivastatin and fluvastatin. These act to decrease cholesterol blood/tissue levels.

The statins have also recently been reported to have potential utility in the treatment of dementia (The Lancet, 2000: 356; 1627-1631) and various cancers, eg. prostate, skin, lung colon, bladder, uterus and kidney (Arch. Intern. Med. 2000, 160: 2363-2368).

However, there are a number of potentially serious side effects associated with statin therapy, including rhabdomyolysis, headache, joint pain, fever, muscle pain, back pain, abdominal cramping, sleep disorder, rhinitis, sinusitis, stimulation of coughing reflex, dizziness and fatigue. Of the contraindications for this group of drugs, two of the most common are fatigue and/or muscle pain (often referred to as "myalgia"). In severe cases, these symptoms may lead to the undesirable cessation of the vital therapy. In rare cases, severe muscle wastage (rhabdomyolysis) has been reported. The risk of adverse side effects during treatment with the statins is increased with concurrent administration of certain other drugs, such as cyclosporin, fibric acid derivatives (eg. gemfibrozii), erthyromycin, niacin or other antifungals. Similar symptoms to those experienced by patients undergoing statin therapy may also be experienced by patients undergoing therapy with other drugs, or may be experienced as a result of a disease state.

Thus, there exists a need for the treatment of muscle pain and fatigue generally and especially for treatment of side effects associated with certain drug therapies, particularly the side effects associated with statin therapy.

It has now been found that certain substituted benzoquinones, such as Coenzymes Q, particularly Q10, can be used in treating muscle pain and fatigue and for treating adverse side effects associated with some drug therapies. In particular, reversal or prevention of adverse statin therapy related side effects can be achieved by administering certain substituted benzoquinones simultaneously, sequentially or separately to administration of uridine, certain of its biological precursors or a salt, ester or tautomer thereof. These compounds can therefore provide a useful adjunctive therapy to certain drug therapies.

US5316765, EP0383432 and US4929437 disclose the use of ubiquinone Q10 (the preferred compound of formula (I)) to treat the side effects of statin therapy; W09316704 the use of ubiquinone Q10 to treat the side effect of cancer treatment.

BE1005939, DE2462312 and DATABASE WPI Section Ch, Week 198345 Derwent Publications Ltd., London, GB; Class B03, AN 1983-813471 XP002311568 & SU 988 814 A (ORDZHONIKIDZE CHEM-PHARM) 15 January 1983 disclose the use of orotic acid for the treatment of conditions like fatigue under physical stress, fatigue, migraine. However, these documents do not mention in any way the treatment of side effects of drug administration. From these documents it is not possible to predict whether orotic acid could be effective to treat these specific symptoms, let alone its use in combination with compounds of formula (I).

### SUMMARY OF THE INVENTION

According to one embodiment of the present invention there is provided use of uridine, one of its biological precursors selected from uridine monophosphate, uridine triphosphate, orotic acid, dihydroorotate, triacetyl uridine and N-carbamoylaspartate, or a salt, ester or tautomer thereof and at least one compound of Formula (I) 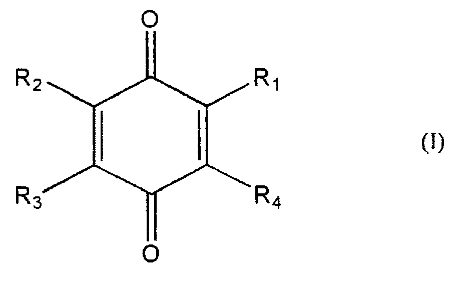 wherein
R₁ is selected from H or C₁₋₁₆ alkyl;
R₂ and R₃ are each independently selected from H, hydroxy, C₁₋₁₆ alkyl, C₁₋₆alkoxy, C₁₋₆ alkenyl, C₁₋₆ alkenoxy, C₁₋₆ alkynyl or C₁₋₆ alkynoxy, and
R₄ is alkyl, alkenyl, alkoxy, alkenoxy, alkylol or alkenylol;
in preparation of a medicament for treatment of one or more side effects of drug therapy.

According to another embodiment of the present invention there is provided a composition comprising uridine, one of its biological precursors selected from uridine monophosphate, uridine triphosphate, orotic acid, dihydroorotate, triacetyl uridine and N-carbamoylaspartate, or a salt, ester or tautomer thereof and at least one compound of Formula (I) 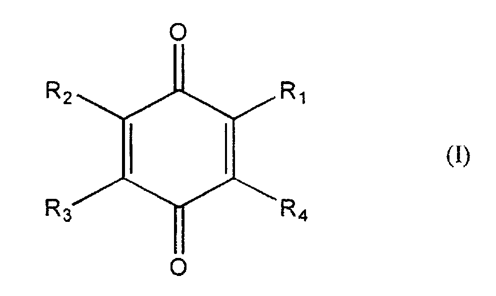 wherein
R₁ is selected from H or C₁₋₁₆ alkyl;
R₂ and R₃ are each independently selected from H, hydroxy, C₁₋₁₆ alkyl, C₁₋₆ alkoxy, C₁₋₆ alkenyl, C₁₋₆ alkenoxy, C₁₋₆ alkynyl or C₁₋₆ alkynoxy; and
R₄ is alkyl, alkenyl; alkoxy, alkenoxy, alkylol or alkenylol ;
and at least one statin, preferably the at least one statin is selected from atorvastatin, simvastatin, pravastatin, lovastatin, cerivastatin and fluvastatin.

In a further embodiment of the present invention there is provided a combination pack or kit comprising uridine, one of its biological precursors selected from uridine monophosphate, uridine triphosphate, orotic acid, dihydroorotate, triacetyl uridine and N-carbamoylaspartate, or a salt, ester or tautomer thereof and at least one compound of Formula (I) 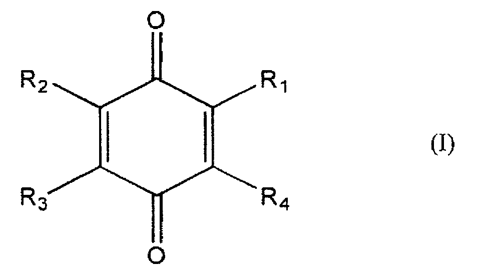 wherein
R₁ is selected from H or C₁₋₁₆ alkyl;
R₂ and R₃ are each independently selected from H, hydroxy C₁₋₁₆ alkyl, C₁₋₆ alkoxy, C₁₋₆ alkenyl, C₁₋₆ alkenoxy, C₁₋₆alkynyl or C₁₋₆ alkynoxy; and
R₄ is alkyl, alkenyl, alkoxy, alkenoxy, alkylol or alkenylol;
and at least one statin, preferably the at least one statin is selected from atorvastatin, simvastatin, pravastatin, lovastatin, cerivastatin and fluvastatin, wherein said pack or kit is adapted for the simultaneous, sequential or separate administration of the statin, uridine, one of its biological precursors or a salt, ester or tautomer thereof and the compound of Formula (I).

### BRIEF DESCRIPTION OF THE FIGURES

Figure i graphically depicts the increasing absence of muscle pain in a patient taking Q 10 as determined over a 30 day period.

### DETAILED DESCRIPTION OF THE INVENTION

Throughout this specification and the claims which follow, unless the context requires otherwise, the word "comprise" and variations such as "comprises" and "comprising" will be understood to imply the inclusion of a stated integer or step or group of integers but not the exclusion of any other integer or step or group of integers.

As used herein, the term alkyl refers to straight chain or branched cyclic fully saturated hydrocarbon residues, preferably straight chain or branched alkyl. Examples of straight chain and branched alkyl include C₁₋₂₀ alkyl such as methyl, ethyl, propyl, isopropyl, butyl, isobutyl, see-butyl, tert-butyl, amyl, isoamyl, sec-amyl, 1,2-dimethylpropyl, 1,1-dimethylpropyl, hexyl, 4-methylpentyl, 1-methylpentyl, 2-methylpentyl, 3-methylpentyl, 1,1-dimethylbutyl, 2,2-dimethylbutyl, 3,3-dimethylbutyl, 1,2-dimethylbutyl, 1,3-dimethylbutyl, 1,2,2,-trimethylpropyl, 1,1,2- trimethylpropyl, heptyl, 5-methylhexyl, 1-methylhexyl, 2,2-dimethylpentyl, 3,3-dimethylpentyl, 4,4-dimethylpentyl, 1,2-dimethylpentyl, 1,3-dimethylpentyl 1,4-dimethyl-pentyl, 1,2,3,-trimethylbutyl, 1,1,2-trimethylbutyl, 1,1,3,-trimethylburyl, octyl, 6-methylheptyl, 1-methylheptyl, 1,1,3,3-tetramethylbutyl, nonyl, 1-, 2-, 3-, 4-, 5-, 6- or 7-methyl-octyl, 1-, 2-, 3-, 4- or 5-ethylheptyl, 1-, 2- or 3-propylhexyl, decyl, 1-, 2-, 3-, 4-, 5-, 6-, 7- and 8-methylnonyl, 1-, 2-, 3-, 4-, 5- or 6-ethyloctyl, 1-, 2-, 3- or 4-propylheptyl, undecyl, 1-, 2-, 3-, 4-, 5-, 6-, 7-, 8- or 9-methyldecyl, 1-, 2-, 3-, 4-, 5-, 6- or 7-ethylnonyl, 1-, 2-, 3-, 4- or 5-propylocytl, 1-, 2- or 3-butylheptyl, 1-pentylhexyl, dodecyl, 1-, 2-, 3-, 4-, 5-, 6-, 7-, 8-, 9- or 10-methylundecyl, 1-, 2-, 3-, 4-, 5-, 6-, 7- or 8-ethyldecyl, 1-, 2-, 3-, 4-, 5- or 6-propylnonyl, 1-, 2-, 3- or 4-butyloctyl, 1-2-pentylheptyl and the like. Other examples of alkyl include C₂₁₋₂₅ alkyl, C₂₆₋₃₀ alkyl, C₃₁₋₃₅ alkyl, C₃₆₋₄₀ alkyl, C₄₁₋₄₆ alkyl, C₅₀-₅₅ alkyl and C₅₆₋₆₀ alkyl. Examples of cyclic alkyl include mono- or polycyclic alkyl groups such as cyclopropyl, cyclobutyl, cyclopentyl, cyclohexyl, cycloheptyl, cyclooctyl, cyclononyl, cyclodecyl and the like.

As used herein the term "alkenyl" denotes groups formed from straight chain, branched or cyclic hydrocarbon residues containing at least one carbon-carbon double bond including ethylenically mono-, di- or poly-unsaturated alkyl or cycloalkyl groups as previously defined. Examples of alkenyl include C₁₋₂₀ alkenyl such as vinyl, allyl, 1-methylvinyl, butenyl, iso-butenyl, 3-methyl-2-butenyl, 1-pentenyl, cyclopentenyl, 1-methyl-cyclopentenyl, 1-hexenyl, 3-hexenyl, cyclohexenyl, 1-heptenyl, 3-heptenyl, 1-octenyl, cyclooctenyl, 1-nonenyl, 2-nonenyl, 3-nonenyl, 1-decenyl, 3-decenyl, 1,3-butadienyl, 1-4,pentadienyl, 1,3-cyclopentadienyl, 1,3-hexadienyl, 1,4-hexadienyl, 1,3-cyclohexadienyl, 1,4-cyclohexadienyl, 1,3-cycloheptadienyl, 1,3,5-cycloheptatrienyl and 1,3,5,7-cyclooctatetraenyl.

Other examples of alkenyl groups include C₁₀-C₁₅ alkenyl, C₁₆-C₂₀ alkenyl, C₂₁-C₂₅ alkenyl, C₂₆-C₃₀ alkenyl, C₃₁-C₃₅ alkenyl, C₃₆-C₄₀ alkenyl, C₄₁-C₄₅ alkenyl, C₄₆-C₅₀ alkenyl, C₅₁-C₅₅ alkenyl, C₅₆-C₆₀ alkenyl, C₆₁₋₆₅ alkenyl, C₆₆₋₇₀ alkenyl and C₇₁₋₈₀ alkenyl. Each of these may contain one or more alkyl or alkenyl branches.

Particular examples of alkenyl include:
- isoprenoid chains of formula (a): 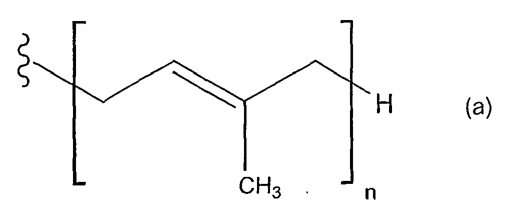
where n = 1-20
(poly)alkenyl chains of formula (b), (c), (d), (e) or (f): 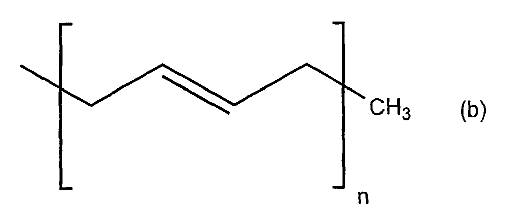 where n = 1-20 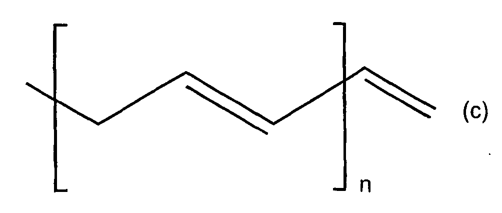 where n = 1-20 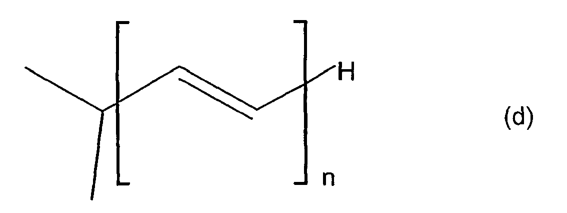 where n=1-20 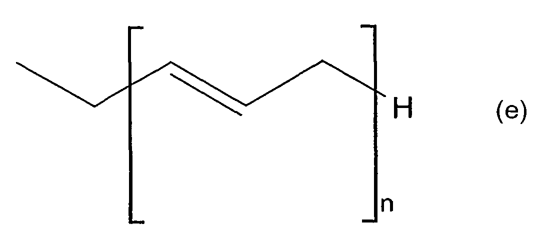 where n=1-20; and 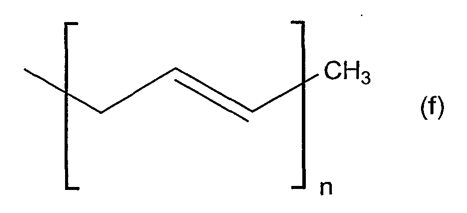 wherein n=1-20.

As used herein the term "alkynyl" denotes groups formed from straight chain, branched or cyclic hydrocarbon residues containing at least one carbon-carbon triple bond including ethynically mono-, di- or poly- unsaturated alkyl or cycloalkyl groups as previously defined. Unless the number of carbon atoms is specified the term preferably refers to C₁₋₂₀ alkynyl. Examples include ethynyl, 1-propynyl, 2-propynyl, butynyl isomers and pentynyl isomers.

The terms "alkoxy", "alkenoxy" and "alkynoxy" respectively denote alkyl, alkenyl and alkynyl groups as hereinbefore defined when linked by oxygen. The terms "alkylol" and "alkenylol" denote alkyl and alkenyl groups, respectively, substituted in one or more positions by hydroxyl.

It will be appreciated that one or more compounds of formula (I) or other compounds of the invention can have an asymmetric centre and therefore can exist in more than one stereoisomeric form. The invention extends to each of these forms individually and to mixtures thereof, including racemates.

In a preferred form of the invention, in Formula (I) R₁ is hydrogen, methyl, ethyl or propyl, preferably hydrogen or methyl.

In another preferred form, R₂ and R₃ are independently selected from H, C₁₋₆ alkyl or C₁₋₆ alkoxy, particularly H, methyl, ethyl, propyl, methoxy, ethoxy or propoxy, preferably H, methyl or methoxy. In another preferred form R₂ and R₃ are the same and may both be H, methyl or methoxy.

In yet another preferred form of the invention, R₄ is an isoprenoid side chain of formula (a). Particularly preferred is a side chain of formula (a) wherein n is 6 to 10. A preferred form is where n is 10.

Particularly preferred compounds are those where R₁ is hydrogen or methyl, and R₂ and R₃ are both hydrogen or methyl or methoxy. Particularly preferred compounds are those where R₁ is methyl and R₂ and R₃ are both methoxy.

Another preferred class of compounds of Formula (I) are the Coenzyme Q compounds, also known as the ubiquinones, which include Coenzymes Q6, Q7, Q8, Q9, Q 10 and Q11. A particularly preferred compound is Coenzyme Q10, which may also be referred to as Coenzyme Q₁₀ and which will be referred to herein as Q10.

Compounds of Formula (I) may be commercially available (eg Q10) or may be synthesised using methods known in organic chemistry or obtained by microbiological means or may be derived from compounds obtained by any one or more of these means.

By the phrase "uridine, its biological precursors or a salt, ester, tautomer thereof' it is intended to embrace uridine and compounds which upon administration to a human or animal would be converted *in vivo* to uridine or to a compound having equivalent activity within the human or animal system to uridine. *In vivo* conversion to uridine or to a compound having equivalent activity to undine may involve one or more chemical conversion steps. For convenience, throughout this specification this class of compounds will be referred to as "uridine related compounds". Clearly, within this class there are a number of subclasses of undine related compounds including biological precursors of undine or salts, esters, tautomers or analogues of these biological precursors. As would be well understood by a skilled person the term "biological precursor" is intended to define compounds would be converted over one or more steps to uridine within a human or animal system. Preferably the conversion will be over one to four steps, preferably one or two steps. In this invention the biological precursors of uridine are uridine monophosphate, uridine triphosphate, orotic acid, dihydroorotate, triacetyl uridine and N-carbamoylaspartate. Salts of such compounds with biologically acceptable cations such as ions of magnesium, sodium, potassium, as well as tautomers, such as keto-enol tautomers and esters of such compounds are also embraced by the invention. A particularly preferred salt of orotic acid is magnesium orotate.

The methods and compositions of the invention may be used to treat humans, mammals or other animal subjects. The invention is considered to be particularly suitable for the treatment of human subjects. Non-human subjects may include primates, livestock animals, domestic companion animals and laboratory test animals.

The compounds of Formula (I) are administered in a treatment effective amount. Reference herein to a treatment effective amount is intended to include an amount which, when administered according to a desired dosing regimen, will at least partially attain the desired therapeutic effect or will inhibit, halt or otherwise delay the onset of fatigue, muscle pain or a side effect of the drug treatment concerned. The term "treatment" therefore embraces prophylactic treatments.

Dosing may occur at intervals of hours, days or weeks and may be continued for as long as the desired therapeutic effect is maintained or required. Suitable dosages and dosing regimens can be determined by an appropriate health professional and may depend on the particular cause of the side effect, the severity of the condition as well as the general health, age and weight of the subject.

Suitable dosages of compounds of Formula (I) may lie within the range of 10 mg to 4000 mg per day (ie. per 24 hour period), such as 50 to 2000 mg per day. Particularly suitable dosages may lie in the range of 100 to 1000 mg per day. Preferably the compounds of Formula (I) are administered from once to four times per day. Some exemplary administration regimes are as follows: 1 x 200 mg, 1 x 250 mg, 1 x 300mg or 1 x 400mg per day, or twice a day, eg 2 x 100 mg, 2 x 150 mg or 2 x 200 mg Dosage forms may be of any suitable size (eg 10mg, 50 mg or 100mg). In one preferred embodiment of the invention Q10 is administered twice a day as two doses each of 150mg (which could for example comprise 3 x 50mg soft gel capsules) to give a total administration of 300mg per day.

Suitable dosages of uridine related compounds may lie within the range of 10 mg to 10g per day, such as 500 to 5 g per day. Particularly suitable dosages may lie in the range of 1000 to 4000 mg per day. Preferably the uridine or related compounds are administered from once to four times per day. Some exemplary administration regimes are as follows: 1 x 800 mg, 1 x 1200 mg, 1 x 1600mg or 1 x 2000mg per day, or twice a day, eg 2 x 400 mg, 2 x 600 mg, 2 x 800mg or 2 x 1000 mg. Dosage forms may be of any suitable size (eg 200mg, 400 mg or 1000mg). In one preferred embodiment of the invention magnesium orotate is administered twice a day as two doses each of 800mg (which could for example comprise 2 x 400mg tablets) to give a total administration of 1600mg per day.

The compositions of the invention may be used to treat any type of muscle fatigue or pain arising from certain conditions or diseases, surgery, injury or as a side effect of certain drug therapies. Muscle pain and/or fatigue associated with conditions or diseases such as CFS, fibromyalgia, myofascial pain syndrome, viral infections, myolysis, rhabdomyolysis and neuromuscular diseases may also be treated by the compounds of Formula (I), preferably in conjunction with uridine related compounds.

One example of a group of therapeutic drugs characterised by side effects treatable by the present invention is the statins, of which some notable examples are atorvastatin, simvastatin, pravastatin, lovastatin, cerivastatin and fluvastatin. As indicated above, common side effects associated with statin therapy include rhabdomyolysis, headache, joint pain, fever, muscle pain, back pain, abdominal cramping, sleep disorder, rhinitis, sinusitis, stimulation of coughing reflex, dizziness and fatigue. Other examples of therapeutic drugs for which muscle fatigue and/or pain or other symptoms treatable by the inventive methods may be a side effect are AZT, hypercholesterolemia therapy drugs (apart from the statins, such as bile acid binding agents such as cholestyramine and colestipol or others such as niacin, probucol or HMG-CoA reductase inhibitors which are not statins), hyperlipidemia therapy drugs, corticosteroids and cancer chemotherapy drugs. Other specific examples of drugs which may give rise to side effects treatable by methods of the present invention are gemfibrozil, fenofibrate, ciprofibrate, bezafibrate, betamethasone, cortisone, prednisolone, dexamethasone, hydrocortisone, methylprednisolone, adriamycin, bleomycin, dactinomycin, daunorubicin, doxorubicin, fludarabine, mitozantrone, epirubicin, tamoxifen, goserelin, carboplatin, cisplatin and etoposide or their salts, analogues or derivatives. Thus, the compounds of Formula (I), particularly Q10, preferably combined with uridine related compounds, may be a useful adjunctive treatment where drugs such as the statins, or others are used to treat, for example, AIDS, hypercholesterolemia, hyperlipidemia, dementia or cancers such as prostate, skin, lung, breast, colon, bladder, uterus and kidney cancers.

The at least one compound of Formula (I) may also be administered in conjunction (either separately, simultaneously or sequentially) with other active agents and in particular with one or more further anti-oxidant compound or compounds, such as Vitamin C or E, carotenoids or carnitine, or their derivatives or analogues.

A compound of Formula (I) can be administered alone or in combination with a uridine related compound and/or in conjunction with the therapeutic drug (eg a statin compound) and optionally with a further active agent or anti-oxidant. The combination of components constituting the treatment may be administered either simultaneously (as discrete dosage forms or as a single composition), sequentially, or separated by a suitable time interval. Where the components are administered as discrete dosage forms, ie not as intimate compositions, each component may be administered in the same form or a different form, eg oral, nasal, parenteral, rectal, vaginal or dermal. When the compounds are administered simultaneously, sequentially or separately, the components may be provided as discrete dosage forms. Optionally the components of the combination may be provided in a kit form wherein the kit is preferably in compartmentalised form adapted for the discrete administration of the components.

Alternatively, when the components of the combination are administered simultaneously, they may be provided as a single composition containing the two or more components or may be provided in a kit form, wherein the kit is compartmentalised for the simultaneous administration of the components.

Where the compound of Formula (I), uridine related compound and/or anti-oxidant or other active agent, and/or the therapeutic drug are administered as discrete dosage forms, each may be formulated together with one or more pharmaceutically acceptable additives to form compositions. Where the components of the therapy are administered as a single composition, the composition may also optionally comprise one or more pharmaceutically acceptable additives.

The formulation of pharmaceutical compositions is well known to those skilled in the art. Such compositions may contain any suitable additives such as carriers, diluents or excipients, which are pharmaceutically acceptable in the sense of being compatible with the other ingredients of the composition and not injurious to the subject. Suitable additives include all conventional solvents, oils, dispersion media, fillers, solid carriers, coatings, antifungal and antibacterial agents, dermal penetration agents (where appropriate), surfactants, isotonic and absorption agents and the like. It will be understood that the compositions of the invention may also include other supplementary physiologically active agents. Further details of pharmaceutically acceptable additives may be found in Remington's Pharmaceutical Sciences, 18th Edition, Mack Publishing Co., Easton, Pennsylvania, USA, the disclosure of which is included herein in its entirety by way of reference.

The compositions may conveniently be presented in unit dosage form and may be prepared by methods well known in the art of pharmacy. Such methods include the step of bringing into association the active ingredient with the carrier, which constitutes one or more accessory ingredients. In general, the compositions are prepared by uniformly and intimately bringing into association the active ingredient with liquid carriers or finely divided solid carriers or both, and then if necessary shaping the product.

The compounds and compositions of the invention may be presented for oral administration (although other forms such as parenteral, rectal, vaginal and dermal, may, under appropriate circumstances also be contemplated) and may be presented as discrete units such as capsules, sachets of powders or granules or tablets each containing a predetermined amount of the active ingredient; as a powder or granules; as a solution or a suspension in an aqueous or non-aqueous liquid; oils; paste; or as an oil-in-water liquid emulsion or a water-in-oil liquid emulsion. A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g inert diluent, preservative disintegrant (e.g. sodium starch glycolate, cross-linked polyvinyl pyrrolidone, cross-linked sodium carboxymethyl cellulose) surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach. The compounds may also be presented in the form of hard or soft gelatin capsules

It should be understood that in addition to the active ingredients particularly mentioned above, the compositions of this invention may include other agents or additives conventional in the art having regard to the type of composition in question, for example, those suitable for oral administration may include such further agents as binders, sweeteners, thickeners, flavouring agents disintegrating agents, coating agents, preservatives, lubricants and/or time delay agents. Suitable sweeteners include sucrose, lactose, glucose, aspartame or saccharine. Suitable disintegrating agents include corn starch, methylcellulose, polyvinylpyrrolidone, xanthan gum, bentonite, alginic acid or agar. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl monostearate or glyceryl distearate.

The compounds of the invention may also be presented for use in veterinary compositions. These may be prepared by any suitable means known in the art. Examples of such compositions include those adapted for:
(a) oral administration, external application (eg drenches including aqueous and non-aqueous solutions or suspensions), tablets, boluses, powders, granules, pellets for admixture with feedstuffs, pastes for application to the tongue ;
(b) parenteral administration, eg subcutaneous, intramuscular or intravenous injection as a sterile solution or suspension
(c) topical application eg creams, ointments, gels, lotions etc.

Those skilled in the art will appreciate that the invention described herein is susceptible to variations and modifications other than those specifically described. It is to be understood that the invention includes all such variations and modifications which fall within the spirit and scope of this general description. The invention also includes all of the steps, features, compositions and compounds referred to or indicated in this specification, individually or collectively, and any and all combinations of any two or more of said steps or features.

The invention will now be described with reference to the following examples which are intended for the purpose of illustration only and are not intended to limit the generality hereinbefore described.

### EXAMPLES

### Example 1

The patient, a white male aged approximately 54 years, had suffered for a number of years from unexplained muscle pain which started in the legs and gradually spread to other skeletal muscles. During the worst of the symptoms, the patient was unable to raise his arms above his head and was unable to drive a car. Walking was also difficult.

The patient was prescribed anti-inflammatory drugs and pain killers which did not provide significant relief. The patient was eventually diagnosed as suffering from a number of viral infections: cytomegalovirus (CMV), Epstein-Barr virus (EBV) and hepatitis A.

The patient was started on a regimen of 150 mg per day of Coenzyme Q10. Within several days the patient reported that the muscle pain had subsided noticeably and his general sense of well being has improved. The improvement was reported as permanent provided that the patient kept taking Q10. On one occasion, the patient stopped taking the Q10 and the muscle pain returned. When the patient recommenced the Q10 treatment, the pain diminished within a short period.

### Example 2

The patient, a white female aged approximately 44 years, had suffered for a number of years from chronic fatigue syndrome. The condition was severe enough such that she was unable to pursue her teaching career and day to day tasks were generally performed with a general sense of fatigue and muscle pain. Muscle pain was generally controlled with over the counter (ie. non-prescription) analgesics containing 500 mg paracetamol and 8 mg codeine phosphate, 2- 4 tablets per day.

Table 1 provides a summary of the patient's self assessed levels of general sense of well being, ability to perform day to day tasks and level of (or relative absence of) muscle pain during a dosing regimen of 2 x 50 mg per day of Q10 (50mg taken each at breakfast and dinner). Levels were rated by the patient on a scale of 0-10, with 0 representing severe incapacity or pain and 10 representing the complete absence of pain or fatigue/excellent performance.

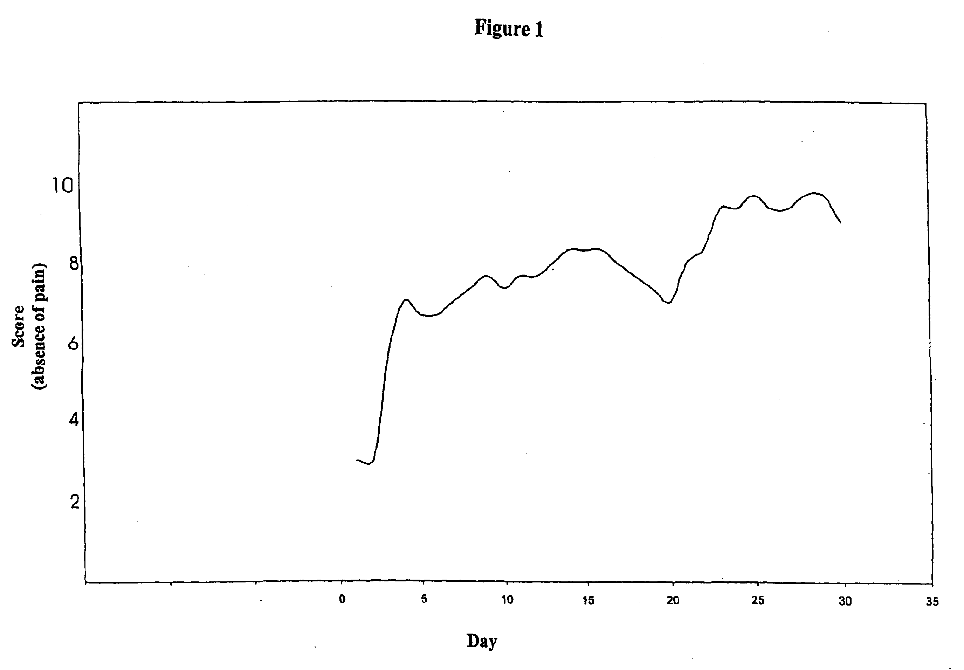

Figure 1 depicts the self assessed (absence of) pain levels for the patient, over a period of 30 days, on a scale of 1-10, where 0 is severe pain and 10 relates to an absence of pain (wellness). On day 1 the patient commenced taking 100 mg of Q10 per day until day 10 when the dosage was increased to 200 mg per day

**Table 1**

| | Day 1 | Day 2 | Day 3 | Day 4 | Day 5 | Week 2 | Week 12 |
|---|---|---|---|---|---|---|---|
| Absence of Muscle Pain | 3* | 3** | 7 | 7 | 7 | 7 | 9 |
| Sedentary Work | 3 | 3 | 4 | 5 | 6 | 7 | 9 |
| Light Work/Walking | 4 | 4 | 6 | 6 | 7 | 7 | 9 |
| Work: teacher | 0 | 0 | 0 | 0 | 0 | 3 | 8 |
| Heavy Work (1 hour) | 0 | 0 | 3*** | 3*** | 3*** | 5 | 8 |
| Feeling of Well Being | 4 | 4 | 5 | 6 | 7 | 7 | 9 |
| Wellness Score (out of 60) | 14 | 14 | 25 | 27 | 30 | 36 | 52 |

| | | | | | | | |
|---|---|---|---|---|---|---|---|
| * patient required 2x analgesic tablets to control pain ** patient required 2x2 (4) x analgesic tablets to control pain *** patient performed only ½ hour heavy work. | | | | | | | |

### Example 3

### Pilot Clinical Study - Administration of Coenzyme Q10 to patients suffering from statin induced fatigue and muscle pain

Table 2 outlines data for four patients (numbered #1-#4) undergoing statin therapy for treatment of hypercholesterolemia and who had reported suffering from varying levels of muscular pain and fatigue. This study is ongoing, but the results below follow the study with these four patients from week minus 1 (WK(-1)) to week plus 4 (WK(+4)).

Patients were either contacted or attended the clinic at weeks -1, 0, +1, +2 and +4. At the clinic on the first week (WK(-1)) details of the patients' statin medication were recorded and daily doses were noted. Patients were also scored for pain using the McGill Pain Questionnaire (Melzack, R., 1975 "The McGill Pain Questionnaire: Major Properties and Scoring Methods". Pain 1:277-299, the disclosure of which is included herein in its entirety by way of reference) scales for Pain Rating Index (PRI) and Present Pain Intensity (PPI). In the PRI index, which comprises two scores, higher scores indicate increasing levels of pain. In the PPI index present pain is given a score of 0 to 5, where 0 represents no pain and 5 represents excruciating pain. Patients were also scored at WK(-1) for fatigue using the Fatigue Impact Scale (Fisk, J.D. et al, 1994, "Measuring the functional impact of fatigue: Initial Validation of the Fatigue Impact Scale", Clinical Infectious Disease, 18 (Suppl 1):S79-83, the disclosure of which is included herein in its entirety by way of reference). Questionnaires to determine PRI, PPI and FIS scores were conducted thereafter at weeks +1, +2 and +4. At weeks +1 and +2 the patients were contacted by telephone and were asked to mail their self assessment questionnaires to the clinic.

At WK(0) and WK(+4) blood samples were taken from patients to determine individual baseline levels of Q10 (Q10) (µg/ml) (to monitor patient compliance), creatine kinase concentration (CK) (units/L) (as a blood measure of muscle trauma) and alanine aminotransferase concentration (ALT) (units/L) (as a measure of liver damage), as well as serum lipids levels. In patients #1 - #4 the administration of Q10 did not significantly affect statin therapy with respect to serum cholesterol, HDL-cholesterol or LDL-cholesterol and triglyceride levels.

It is to be noted that the normal range for blood creatine kinase concentration is 0-200 units/L and the normal range for blood concentration of alanine aminotransferase is 0-40 units/L.

Commencing at WK(0) and continuing through the study the patients were asked to take a daily dose of 300 mg of Coenzyme Q10 (Q10), which was administered as 3 x 50mg soft gel capsules (commercially available from R.P. Scherer), morning and evening.

The results of the parameters mentioned above are shown in Table 2. Marginal decreases in PRI and FIS scores have been noted for a few patients, although these decreases have not been of great significance. It is possible that patient pain and fatigue measures may decrease with prolonged treatment.

**TABLE 2 - Q10 administration**

| | | PRI | | | | | | | | PPI | | | | FIS | | | | Q10 (µg/ml) | | CK (units/L) | | ALT (units/L) | |
|---|---|---|---|---|---|---|---|---|---|---|---|---|---|---|---|---|---|---|---|---|---|---|---|
| Patient | Statin (mg/day) | WK(-1) | | WK(+1) | | WK(+2) | | WK(+4) | | WK (-1) | WK (+1) | WK (+2) | WK (+4) | WK (-1) | WK (+1) | WK (+2) | WK (+4) | WK (0) | WK (+4) | WK (0) | WK (+4) | WK (0) | WK (+4) |
| #1 | Simvastatin | 12 | 5 | 19 | 7 | 10 | 6 | 9 | 5 | 2 | 1 | 2 | 2 | 15 | 18 | 16 | 16 | * | 2.22 | 160 | 148 | 31 | 25 |
| | 20 | | | | | | | | | | | | | | | | | | | | | | |
| #2 | Lipitor | 15 | 5 | 22 | 10 | 25 | 11 | 8 | 4 | 2 | 2 | 2 | 2 | 48 | 42 | 47 | 43 | 0.59 | 2.42 | 175 | 127 | ** | 11 |
| | 40 | | | | | | | | | | | | | | | | | | | | | | |
| #3 | Lipitor | 10 | 3 | 0 | 0 | 9 | 6 | 4 | 1 | 2 | 0 | 1 | 1 | 39 | 19 | 44 | 6 | 0.46 | 2.49 | 69 | 78 | 14 | 19 |
| | 10 | | | | | | | | | | | | | | | | | | | | | | |
| #4 | Lipitor | 69 | 30 | 48 | 21 | 54 | 23 | 46 | 20 | 2 | 3 | 2 | 2 | 104 | 91 | 90 | 90 | 1.39 | 1.92 | 39 | 32 | 33 | 35 |
| | 20 | | | | | | | | | | | | | | | | | | | | | | |

| | | | | | | | | | | | | | | | | | | | | | | | |
|---|---|---|---|---|---|---|---|---|---|---|---|---|---|---|---|---|---|---|---|---|---|---|---|
| * Data not available. Sample of 600 healthy volunteer subjects has demonstrated that blood Q10 concentration of 2.22 µg/ml is elevated and indicates patient compliance with Q10 administration. ** Data not available. | | | | | | | | | | | | | | | | | | | | | | | |

### Example 4

### Pilot Clinical Study - Administration of Coenzyme Q10 and magnesium orotate to patients suffering from statin induced fatigue and muscle pain

Table 3 outlines data for four patients (numbered #i - #iv) undergoing statin therapy for treatment of hypercholesterolemia and who had reported suffering from varying levels of muscular pain and fatigue. This study is ongoing but the results below follow the study in relation to these four patients from week minus 1 (WK(-1)) to week plus 4 (WK(+4)).

Patients were either contacted or attended the clinic at weeks -1, 0, +1, +2 and +4. On the first week at the clinic (WK(-1)) details of the patients' statin medication were recorded and daily doses noted. Patients were also scored for pain using the McGill Pain Questioimaire (Melzack, R., 1975 "The McGill Pain Questionnaire: Major Properties and Scoring Methods". Pain 1:277-299, the disclosure of which is included herein in its entirety by way of reference) scales for Pain Rating Index (PRI) and Present Pain Intensity (PPI). In the PRI index, which comprises two scores, higher scores indicate increasing levels of pain. In the PPI index present pain is given a score of 0 to 5, where 0 represents no pain and 5 represents excruciating pain. Patients were also scored at WK(-1) for fatigue using the Fatigue Impact Scale (Fisk, J.D. et al, 1994, "Measuring the functional impact of fatigue: Initial Validation of the Fatigue Impact Scale", Clinical Infectious Disease, 18 (Suppl 1):S79-83, the disclosure of which is included herein in its entirety by way of reference). Questionnaires to determine PRI, PPI and FIS scores were conducted thereafter at weeks +1, +2 and +4. At weeks +1 and +2 the patients were contacted by telephone and were asked to mail their self assessment questionnaires to the clinic.

At WK(0) patients commenced taking daily doses of 300 mg of coenzyme Q10 (as described in example 3) and 1600 mg of magnesium orotate, administered as 2 x 400 mg tablets, morning and evening.

Blood samples were taken at WK(0) and WK(+4) to determine individual baseline levels of Q10 (Q10) (µg/ml) and thereby monitor patient compliance. As with example 3, blood samples taken at WK(0) and WK(+4) were also used to determine creatine kinase concentration (CK) (units/L) and alanine aminotransferase concentration (ALT) (units/L), as well as serum lipid levels. In patients #i - #iv the coadministration conducted did not significantly affect statin therapy with respect to serum cholesterol, HDL-cholesterol or LDL-cholesterol and triglyceride levels.

As can be seen from the results shown in Table 3 significant improvements in PRI and PPI pain scores and FIS fatigue score were recorded in virtually all patients undergoing the combined therapy, which would appear to demonstrate synergistic activity in treating pain and fatigue, resulting from the combined administration of Q10 and magnesium orotate.

It is to be noted that the normal range for blood creatine kinase concentration is 0-200 units/L and the normal range for blood concentration of alanine aminotransferase is 0-40 units/L.

Patient #i exhibited an abnormally high serum creatine kinase level at WK(0), illustrative of muscle trauma (411 units/L). After four weeks combined Q10 and magnesium orotate treatment the CK level fell to lie within the normal range (181 units/L). These results highlight the beneficial effects of the combination therapy on muscle trauma.

Patients #i and #ii exhibited abnormally high serum ALT at WK(0) (42 units/L), illustrative of liver damage. After four weeks combined Q10 and magnesium orotate treatment the ALT levels fell to within the normal range (31, 32 units/L, respectively). These results highlight the beneficial effects of the combined therapy on liver damage.

**TABLE 3 - Q10 and Mg Orotate administration**

| | | PRI | | | | | | | | PPI | | | | FIS | | | | Q10 (µg/ml) | | CK (units/L) | | ALT (units/L) | |
|---|---|---|---|---|---|---|---|---|---|---|---|---|---|---|---|---|---|---|---|---|---|---|---|
| Patient | Statin (mg/day) | WK(-1) | | WK(+1) | | WK(+2) | | WK(+4) | | WK (-1) | WK (+1) | WK (+2) | WK (+4) | WK (-1) | WK (+1) | WK (+2) | WK (+4) | WK (0) | WK (+4) | WK (0) | WK (+4) | WK (0) | WK (+4) |
| #i | Pravachol | 56 | 21 | 11 | 6 | 11 | 6 | 12 | 6 | 3 | 1 | 1 | 2 | 47 | 34 | 18 | 19 | 1.11 | 2.37 | 411 | 181 | 42 | 31 |
| | 40 | | | | | | | | | | | | | | | | | | | | | | |
| #ii | Lipitor | 10 | 5 | 7 | 3 | 3 | 2 | 3 | 2 | 2 | 2 | 1 | | 12 | 8 | 7 | 9 | 0.45 | 4.19 | 88 | 137 | 42 | 32 |
| | 40 | | | | | | | | | | | | | | | | | | | | | | |
| #iii | Simvastatin | 20 | 11 | 7 | 5 | 7 | 4 | 6 | 4 | 2 | 1 | 1 | 1 | 17 | 5 | 8 | 7 | 0.49 | 1.67 | 107 | 88 | 19 | 22 |
| | 40 | | | | | | | | | | | | | | | | | | | | | | |
| #iv | Lipitor | 21 | 5 | 0 | 0 | 0 | 0 | 0 | 0 | 3 | 0 | 0 | 0 | 61 | 52 | 40 | 31 | 0.63 | 3.75 | 129 | 135 | 24 | 29 |
| | 10 | | | | | | | | | | | | | | | | | | | | | | |

## Claims

1. Use of uridine, one of its biological precursors selected from uridine monophosphate, uridine triphosphate, orotic acid, dihydroorotate, triacetyl uridine and N-carbamoylaspartate, or a salt, ester or tautomer thereof and at least one compound of Formula (I) 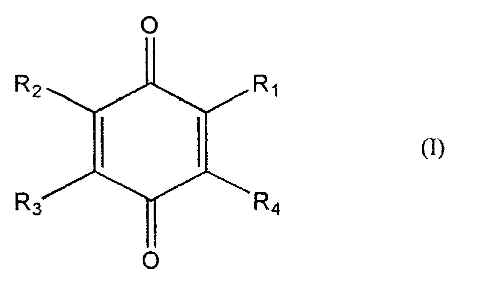 wherein
R₁ is selected from H or C₁₋₁₆ alkyl;
R₂ and R₃ are each independently selected from H, hydroxy, C₁₋₁₆ alkyl. C₁₋₆alkoxy, C₁₋₆ alkenyl, C₁₋₆ alkenoxy, C₁₋₆ alkynyl or C₁₋₆ alkynoxy; and
R₄ is alkyl, alkenyl, alkoxy, alkenoxy, alkylol or alkenylol;
in preparation of a medicament for treatment of one or more side effects of drug therapy.

2. Use according to claim 1, wherein the therapy is for hypercholesterolemia, hyperlipidemia, is a corticosteroid therapy or is a cancer chemotherapy.

3. Use according to claim 2, wherein the drug therapy is statin therapy.

4. The use according to any previous claim wherein said side effect is one or more of rhabdomyolysis, headache, joint pain, fever, muscle pain, back pain, abdominal cramping, sleep disorder, rhinitis, sinusitis, stimulation of coughing reflex, dizziness and fatigue, preferably said side effect is muscle pain.

5. The use according to any previous claim wherein the compound of Formula (I) is Coenzyme Q10.

6. The use according to any preceding claim wherein the uridine precursor is orotic acid or a salt, ester or tautomer thereof; preferably the salt of a uridine precursor is magnesium orotate.

7. Use according to any preceding claim wherein optionally one or more pharmaceutically acceptable additives are used in preparation of a medicament for treatment of one or more side effects of drug therapy.

8. Use according to any preceding claim wherein the medicament further comprises at least one statin, preferably the at least one statin is selected from atorvastatin, simvastatin, pravastatin, lovastatin, cerivastatin and fluvastatin.

9. Use according to any preceding claim wherein the uridine precursor is orotic acid or a salt, ester or tautomer thereof, preferably the salt of a uridine precursor is magnesium orotate.

10. Use according to any preceding claim wherein the medicament is a combination pack or kit wherein said pack or kit is adapted for the simultaneous, sequential or separate administration of the uridine, one of its biological precursors or a salt, ester or tautomer thereof and the compound of Formula (I).

11. Use according to claim 10 in which the combination pack or kit additionally comprises at least one statin, wherein said pack or kit is adapted for the simultaneous, sequential or separate administration of the statin, uridine, one of its biological precursors or a salt, ester or tautomer thereof and the compound of Formula (I).

12. A composition comprising uridine, one of its biological precursors selected from uridine monophosphate, uridine triphosphate, orotic acid, dihydroorotate, triacetyl uridine and N-carbamoylaspartate, or a salt, ester or tautomer thereof and at least one compound of Formula (I) 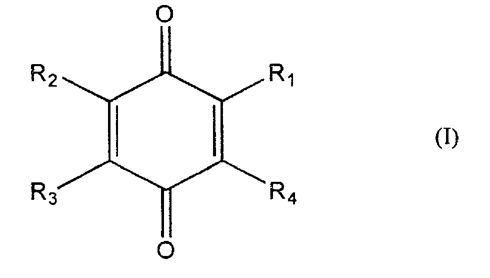 wherein
R₁ is selected from H or C₁₋₁₆ alkyl;
R₂ and R₃ are each independently selected from H, hydroxy, C₁₋₁₆ alkyl, C₁₋₆ alkoxy, C₁₋₆ alkenyl, C₁₋₆ alkenoxy, C₁₋₆ alkynyl or C₁₋₆ alkynoxy; and
R₄ is alkyl, alkenyl, alkoxy, alkenoxy, alkylol or alkenylol;
and at least one statin, preferably the at least one statin is selected from atorvastatin, simvastatin, pravastatin, lovastatin, cerivastatin and fluvastatin.

13. The composition according to claim 12 wherein the compound of Formula (I) is Coenzyme Q10.

14. The composition according to either of claims 12 or 13 wherein the uridine precursor is orotic acid or a salt, ester or tautomer thereof, preferably the salt of a uridine precursor is magnesium orotate.

15. The composition according to any one of claims 12 to 14 further comprising one or more pharmaceutically acceptable additives.

16. Use of a composition according to any of claims 12 to 15 as medicament.

17. A combination pack or kit comprising uridine, one of its biological precursors selected from uridine monophosphate, uridine triphosphate, orotic acid, dihydroorotate, triacetyl uridine and N-carbamoylaspartate, or a salt, ester or tautomer thereof and at least one compound of Formula (I) 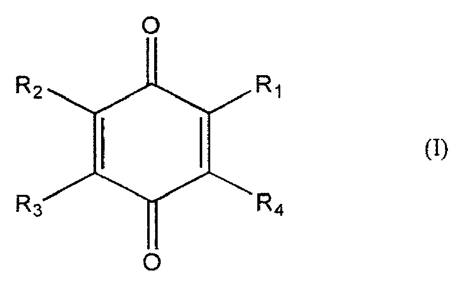 wherein
R₁ is selected from H or C₁₋₁₆ alkyl;
R₂ and R₃ are each independently selected from H, hydroxy, C₁-C₆ alkyl, C₁-C₆ alkoxy, C₁₋₆ alkenyl, C₁₋₆ alkenoxy, C₁₋₆ alkynyl or C₁₋₆ alkynoxy; and
R₄ is alkyl, alkenyl, alkoxy, alkenoxy, alkylol or alkenylol;
and at least one statin, preferably the at least one statin is selected from atorvastatin, simvastatin, pravastatin, lovastatin, cerivastatin and fluvastatin, wherein said pack or kit is adapted for the simultaneous, sequential or separate administration of the statin, uridine, one of its biological precursors or a salt, ester or tautomer thereof and the compound of Formula (I).

## Patentansprüche

1. Verwendung von Uridin, einem seiner biologischen Vorläufer, ausgewählt aus Uridinmonophosphat, Uridintriphosphat, Orotsäure, Dihydroorotat, Triacetyluridin und N-Carbamoylaspartat oder einem Salz, Ester oder Tautomer davon und mindestens einer Verbindung der Formel (1) 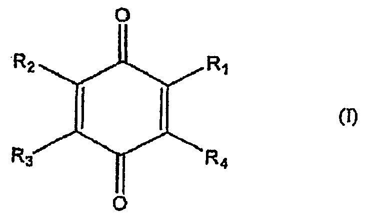 worin
R₁ ausgewählt ist aus H oder C₁₋₁₆Alkyl;
R₂ und R₃ jeweils unabhängig ausgewählt sind aus H, Hydroxy, C₁₋₁₆-Alkyl, C₁₋₆-Alkoxy, C₁₋₆-Alkenyl, C₁₋₆-Alkenoxy, C₁₋₆-Alkinyl oder C₁₋₆-Alkinoxy; und
R₄ Alkyl, Alkenyl, Alkoxy, Alkenoxy, Alkylol oder Alkenylol ist; zur Herstellung eines Medikaments zur Behandlung einer oder mehrerer Nebenwirkungen der Arzneimitteltherapie.

2. Verwendung nach Anspruch 1, worin die Therapie für Hypercholesterinämie, Hyperlipämie ist, eine Coricosteroidtherapie ist oder eine Krebschemotherapie ist.

3. Verwendung nach Anspruch 2, worin die Arzneimitteltherapie eine Statin-Therapie ist.

4. Verwendung nach einem der vorhergehenden Ansprüche, worin die Nebenwirkung eine oder mehrere aus Rhabdomyolose, Kopfschmerz, Gelenkschmerz, Fieber, Muskelschmerz, Rückenschmerz, Abdominalkrampf, Schlafstörung, Rhinitis, Sinusitis, Stimulation von Hustenreflex, Schwindel und Müdigkeit ist, wobei die Nebenwirkung vorzugsweise Muskelschmerz ist.

5. Verwendung nach einem der vorhergehenden Ansprüche, worin die Verbindung der Formel (1) Coenzym Q10 ist.

6. Verwendung nach einem der vorhergehenden Ansprüche, worin der Uridinvorläufer Orotsäure oder ein Salz, Ester oder Tautomer davon ist; wobei das Salz eines Uridinvodäufers vorzugsweise Magnesiumorotat ist.

7. Verwendung nach einem der vorhergehenden Ansprüche, worin optional ein oder mehrere pharmazeutisch verträgliche Additive verwendet werden zur Herstellung eines Medikaments zur Behandlung einer oder mehrerer Nebenwirkungen einer Arzneimitteltherapie.

8. Verwendung nach einem der vorhergehenden Ansprüche, worin das Medikament weiterhin mindestens ein Statin umfasst, wobei das mindestens eine Statin vorzugsweise ausgewählt ist aus Atorvastatin, Simvastatin, Pravastatin, Lovastatin, Cerivastatin und Fluvastatin.

9. Verwendung nach einem der vorhergehenden Ansprüche, worin der Uridinvorläufer Orotsäure oder ein Salz, Ester oder Tautomer davon ist, wobei das Salz eines Uridinvorläufers vorzugsweise Magnesiumorotat ist.

10. Verwendung nach einem der vorhergehenden Ansprüche, worin das Medikament eine Kombinationspackung oder ein Kit ist, worin die Packung oder der Kit angepasst sind zur gleichzeitigen, aufeinanderfolgenden oder getrennten Verabreichung des Uridins, einer seiner biologischen Vorläufer oder eines Salzes, Esters oder Tautomers davon und der Verbindung der Formel (I).

11. Verwendung nach Anspruch 10, worin die Kombinationspackung oder der Kit zusätzlich mindestens ein Statin umfassen, wobei die Packung oder der Kit angepasst sind zur gleichzeitigen, aufeinanderfolgenden oder getrennten Verabreichung des Statins, Uridins, einer seiner biologischen Vorläufer oder eines Salzes, Esters oder Tautomers davon und mindestens einer Verbindung der Formel (1).

12. Zusammensetzung, umfassend Uridin, einen seiner biologischen Vorläufer, ausgewählt aus Uridinmonophosphat, Uridintriphosphat, Orotsäure, Dihydroorotat, Triacetyluridin und N-Carbamoylaspartat, oder einem Salz, Ester oder Tautomer davon und mindestens einer Verbindung der Formel (I) 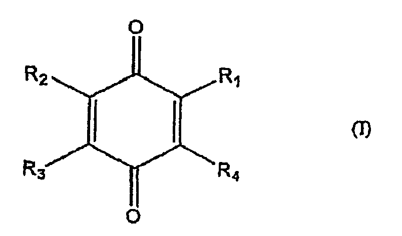 worin
R₁ ausgewählt ist aus H oder C₁₋₁₆-Alkyl;
R₂ und R₃ jeweils unabhängig ausgewählt sind aus H, Hydroxy, C₁₋₁₆-Alkyl, C₁₋₆-Alkoxy, C₁₋₆-Alkenyl, C₁₋₆-Alkenoxy, C₁₋₆-Alkinyl oder C₁₋₆-Alkinoxy; und
R₄ Alkyl, Alkenyl, Alkoxy, Alkenoxy, Alkylol oder Alkenylol ist;
und mindestens ein Statin, wobei das mindestens eine Statin vorzugsweise ausgewählt ist aus Atorvastatin, Simvastatin, Pravastatin, Lovastatin, Cerivastatin und Fluvastatin.

13. Zusammensetzung nach Anspruch 12, worin die Verbindung der Formel (I) Coenzym Q10 ist.

14. Zusammensetzung nach einem der Ansprüche 12 oder 13, worin der Uridinvorläufer Orotsäure oder ein Salz, Ester oder Tautomer davon ist, wobei das Salz eines Uridinvorläufers vorzugsweise Magnesiumorotat ist.

15. Zusammensetzung nach einem der Ansprüche 12 bis 14, weiterhin umfassend ein oder mehrere pharmazeutisch verträgliche Additive.

16. Verwendung einer Zusammensetzung nach einem der Ansprüche 12 bis 15 als ein Medikament.

17. Kombinationspackung oder Kit, umfassend Uridin, einen seiner biologischen Vorläufer, ausgewählt aus Uridinmonophosphat, Uridintriphosphat, Orotsäure, Dihydroorotat, Triacetyluridin und N-Carbamoylaspartat, oder einem Salz, Ester oder Tautomer davon und mindestens eine Verbindung der Formel (I) 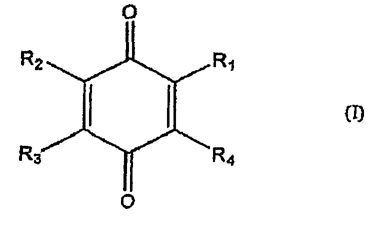 worin
R₁ ausgewählt ist aus H oder C₁₋₁₆-Alkyl;
R₂ und R₃ jeweils unabhängig ausgewählt sind aus H, Hydroxy, C₁₋₁₆-Alkyl, C₁₋₆-Alkoxy, C₁₋₆-Alkenyl, C₁₋₆-Alkenoxy, C₁₋₆-Alkinyl oder C₁₋₆-Alkinoxy; und
R₄ Alkyl, Alkenyl, Alkoxy, Alkenoxy, Alkylol oder Alkenylol ist;
und mindestens ein Statin, wobei das mindestens eine Statin vorzugsweise ausgewählt ist aus Atorvastatin, Simvastatin, Pravastatin, Lovastatin, Cerivastatin und Fluvastatin, wobei die Packung oder der Kit angepasst sind zur gleichzeitigen, aufeinanderfolgenden oder getrennten Verabreichung des Statins, Uridins, einer seiner biologischen Vorläufer oder eines Salzes, Esters oder Tautomers davon und der Verbindung der Formel (I).

## Revendications

1. Utilisation d'uridine, de l'un de ses précurseurs biologiques choisis parmi le monophosphate d'uridine, le triphosphate d'uridine, l'acide orotique, un dihydroorotate, la triacétyl uridine et un N-carbamoylaspartate, ou d'un sel, d'un ester ou d'un tautomère de celui-ci, et d'au moins un composé de formule (I) : 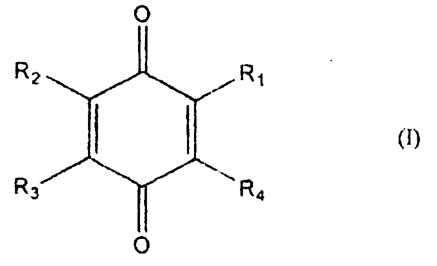 dans laquelle :
R₁ est choisi parmi H ou un groupe alkyle en C₁₋₁₆ ;
R₂ et R₃ sont indépendamment choisis chacun parmi H, un groupe hydroxy, alkyle en C₁₋₁₆, alkoxy en C₁₋₆, alcényle en C₁₋₆, alcénoxy en C₁₋₆, alcynyle en C₁₋₆ ou alcynoxy en C₁₋₆ ; et
R₄ est un groupe alkyle, alcényle, alkoxy, alcénoxy, alkylol ou alcénylol ;
dans la préparation d'un médicament pour le traitement d'un ou plusieurs effets secondaires d'une thérapie par médicament.

2. Utilisation selon la revendication 1, dans laquelle la thérapie est pour l'hypercholestérolémie, l'hyperlipidémie, est une thérapie par corticostéroïde ou est une chimiothérapie de cancer.

3. Utilisation selon la revendication 2, dans laquelle la thérapie par médicament est une thérapie par statine.

4. L'utilisation selon l'une quelconque des revendications précédentes, dans laquelle ledit effet secondaire comprend un ou plusieurs effets choisis parmi la rhabdomyolyse, la migraine, une douleur articulaire, la fièvre, une douleur musculaire, une douleur dorsale, les crampes abdominales, le trouble du sommeil, la rhinite, la sinusite, la stimulation du réflexe de toux, un état vertigineux et la fatigue, ledit effet secondaire étant, de préférence, une douleur musculaire.

5. L'utilisation selon l'une quelconque des revendications précédentes, dans laquelle le composé de formule (I) est la coenzyme Q10.

6. L'utilisation selon l'une quelconque des revendications précédentes, dans laquelle le précurseur d'uridine est l'acide orotique, ou un sel, un ester ou un tautomère de celui-ci ; le sel de précurseur d'uridine étant, de préférence, l'orotate de magnésium.

7. Utilisation selon l'une quelconque des revendications précédentes, dans laquelle on emploie un ou plusieurs additifs pharmaceutiquement acceptables dans la préparation d'un médicament pour le traitement d'un ou plusieurs effets secondaires d'une thérapie par médicament.

8. Utilisation selon l'une quelconque des revendications précédentes, dans laquelle le médicament comprend, en outre, au moins une statine, l'au moins une statine étant, de préférence, choisie parmi l'atorvastatine, la simvastatine, la pravastatine, la lovastatine, la cérivastatine et la fluvastatine.

9. Utilisation selon l'une quelconque des revendications précédentes, dans laquelle le précurseur d'uridine est l'acide orotique ou un sel, un ester ou un tautomère de celui-ci, le sel de précurseur d'uridine étant de préférence l'orotate de magnésium.

10. Utilisation selon l'une quelconque des revendications précédentes, dans laquelle le médicament est un ensemble ou un kit à association, ledit ensemble ou ledit kit étant adapté à l'administration simultanée, séquentielle ou séparée de l'uridine, de l'un de ses précurseurs biologiques, ou d'un sel, d'un ester ou d'un tautomère de celui-ci, et du composé de formule (I).

11. Utilisation selon la revendication 10, dans laquelle l'ensemble ou le kit à association comprend, en outre, au moins une statine, ledit ensemble ou kit étant adapté à l'administration simultanée, séquentielle ou séparée de la statine, d'uridine, de l'un de ses précurseurs biologiques, ou d'un sel, d'un ester ou d'un tautomère de celui-ci, et du composé de formule (I).

12. Composition comprenant de l'uridine, un de ses précurseurs biologiques choisis parmi le monophosphate d'uridine, le triphosphate d'uridine, l'acide orotique, un dihydroorotate, la triacétyl uridine et un N-carbamoylaspartate, ou d'un sel, d'un ester ou d'un tautomère de celui-ci, et d'au moins un composé de formule (1) : 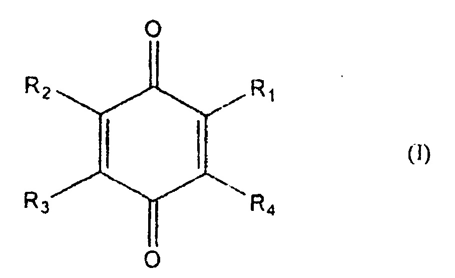 dans laquelle :
R₁ est choisi parmi H ou un groupe alkyle en C₁₋₁₆;
R₂ et R₃ sont indépendamment choisis chacun parmi H, un groupe hydroxy, alkyle en C₁₋₁₆, alkoxy en C₁₋₆, alcényle en C₁₋₆, alcénoxy en C₁₋₆, alcynyle en C₁₋₆ ou alcynoxy en C₁₋₆; et
R₄ est un groupe alkyle, alcényle, alkoxy, alcénoxy, alkylol ou alcénylol ;
et au moins une statine, l'au moins une statine étant, de préférence, choisie parmi l'atorvastatine, la simvastatine, la pravastatine, la lovastatine, la cérivastatine et la fluvastatine.

13. La composition selon la revendication 12, dans laquelle le composé de formule (I) est la coenzyme Q10.

14. La composition selon la revendication 12 ou 13, dans laquelle le précurseur d'uridine est l'acide orotique ou un sel, un ester ou un tautomère de celui-ci, le sel de précurseur d'uridine étant, de préférence, l'orotate de magnésium.

15. La composition selon l'une quelconque des revendications de 12 à 14, comprenant, en outre, un ou plusieurs additifs pharmaceutiquement acceptables.

16. Utilisation d'une composition selon l'une quelconque des revendications 12 à 15, en tant que médicament.

17. Ensemble ou kit à association, comprenant de l'uridine, un de ses précurseurs biologiques choisis parmi le monophosphate d'uridine, le triphosphate d'uridine, l'acide orotique, un dihydroorotate, la triacétyl uridine et un N-carbamoylaspartate, ou d'un sel, d'un ester ou d'un tautomère de celui-ci, et d'au moins un composé de formule (I) : 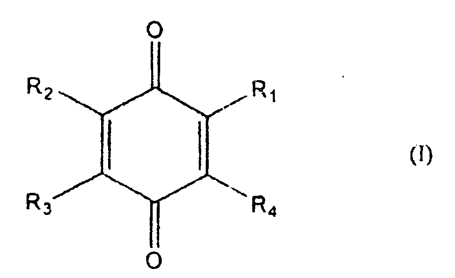 dans laquelle :
R₁ est choisi parmi H ou un groupe alkyle en C₁₋₁₆ ;
R₂ et R₃ sont indépendamment choisis chacun parmi H, un groupe hydroxy, alkyle en C₁₋₁₆, alkoxy en C₁₋₆, alcényle en C₁₋₆, alcénoxy en C₁₋₆, alcynyle en C₁₋₆ ou alcynoxy en C₁₋₆; et
R₄ est un groupe alkyle, alcényle, alkoxy, alcénoxy, alkylol ou alcénylol ;
et au moins une statine, l'au moins une statine étant de préférence choisie parmi l'atorvastatine, la simvastatine, la pravastatine, la lovastatine, la cérivastatine et la fluvastatine, dans lequel ledit ensemble ou kit à association, est adapté à l'administration simultanée, séquentielle ou séparée de la statine, de l'uridine, de l'un de ses précurseurs biologiques, ou d'un sel, d'un ester ou d'un tautomère de celui-ci, et du composé de formule (1).
